# EUROPEAN PATENT APPLICATION

(11) **EP 2 533 052 A1**
(43) Date of publication of application: **12.12.2012**
(21) Application number: 11004636.4
(22) Date of filing: 07.06.2011
(51) Int. Cl.: G01N 33/68, C07K 16/00

(54) **Diagnostic use of proSomatostatin**

(71) Applicant: B.R.A.H.M.S GmbH, 16761 Hennigsdorf (DE)
(72) Inventor: Struck, Joachim, Dr., 13465 Berlin (DE)
(74) Representative: Tomerius, Isabel

(57) **Abstract**

The present invention relates to a method for the diagnosis, prognosis, monitoring and risk assessment of disturbances of the gastrointestinal tract activity and/or function and/or disturbances of the nutritional condition in a patient comprising the steps of: providing a sample of a bodily fluid of a patient; determining the level of proSomatostatin 1-64 or fragments thereof in said sample; correlating the level of proSomatostatin 1-64 or fragments thereof to the diagnosis, prognosis and risk assessment of disturbances of the gastrointestinal tract activity and/or function and/or disturbances of the nutritional condition in said patient, wherein said fragments have a length of at least 6 amino acid residues. The invention also relates to antibodies and a kit containing the antibodies.

SEQUENCE LISTING

<110> B.R.A.H.M.S. GmbH

<120> DIAGNOSTIC USE OF PROSOMATOSTATIN

<130> B.P 712 EP

<160> 6

<170> PatentIn version 3.3

<210> 1
<211> 116
<212> PRT
<213> Human

<400> 1

Met Leu Ser Cys Arg Leu Gln Cys Ala Leu Ala Ala Leu Ser Ile Val
1               5                   10                  15

Leu Ala Leu Gly Cys Val Thr Gly Ala Pro Ser Asp Pro Arg Leu Arg
            20                  25                  30

Gln Phe Leu Gln Lys Ser Leu Ala Ala Ala Ala Gly Lys Gln Glu Leu
        35                  40                  45

Ala Lys Tyr Phe Leu Ala Glu Leu Leu Ser Glu Pro Asn Gln Thr Glu
    50                  55                  60

Asn Asp Ala Leu Glu Pro Glu Asp Leu Ser Gln Ala Ala Glu Gln Asp
65                  70                  75                  80

Glu Met Arg Leu Glu Leu Gln Arg Ser Ala Asn Ser Asn Pro Ala Met
                85                  90                  95

Ala Pro Arg Glu Arg Lys Ala Gly Cys Lys Asn Phe Phe Trp Lys Thr
            100                 105                 110

Phe Thr Ser Cys
        115

<210> 2
<211> 92
<212> PRT
<213> Human

<400> 2

Ala Pro Ser Asp Pro Arg Leu Arg Gln Phe Leu Gln Lys Ser Leu Ala
1               5                   10                  15

Ala Ala Ala Gly Lys Gln Glu Leu Ala Lys Tyr Phe Leu Ala Glu Leu
            20                  25                  30

Leu Ser Glu Pro Asn Gln Thr Glu Asn Asp Ala Leu Glu Pro Glu Asp
        35                  40                  45

Leu Ser Gln Ala Ala Glu Gln Asp Glu Met Arg Leu Glu Leu Gln Arg
    50                  55                  60

Ser Ala Asn Ser Asn Pro Ala Met Ala Pro Arg Glu Arg Lys Ala Gly
65                  70                  75                  80

Cys Lys Asn Phe Phe Trp Lys Thr Phe Thr Ser Cys
                85                  90

<210>  3
<211>  21
<212>  PRT
<213>  Synthetic

<400>  3

Ala Pro Ser Asp Pro Arg Leu Arg Gln Phe Leu Gln Lys Ser Leu Ala
1               5                   10                  15

Ala Ala Ala Gly Lys
            20

<210>  4
<211>  21
<212>  PRT
<213>  Synthetic

<400>  4

Gln Glu Leu Ala Lys Tyr Phe Leu Ala Glu Leu Leu Ser Glu Pro Asn
1               5                   10                  15

Gln Thr Glu Asn Asp
            20

<210>  5
<211>  22
<212>  PRT
<213>  Synthetic

<400>  5

Ala Leu Glu Pro Glu Asp Leu Ser Gln Ala Ala Glu Gln Asp Glu Met
1               5                   10                  15

Arg Leu Glu Leu Gln Arg
            20

<210>  6
<211>  64
<212>  PRT
<213>  Synthetic

<400>  6

Ala Pro Ser Asp Pro Arg Leu Arg Gln Phe Leu Gln Lys Ser Leu Ala
1               5                   10                  15

Ala Ala Ala Gly Lys Gln Glu Leu Ala Lys Tyr Phe Leu Ala Glu Leu
            20                  25                  30

Leu Ser Glu Pro Asn Gln Thr Glu Asn Asp Ala Leu Glu Pro Glu Asp
        35                  40                  45

Leu Ser Gln Ala Ala Glu Gln Asp Glu Met Arg Leu Glu Leu Gln Arg
    50                  55                  60

## Description

### Field of the invention

The present invention relates to the detection of proSomatostatin 1-64 and fragments thereof in biological samples and the use of detecting proSomatostatin 1-64 and fragments thereof for the diagnosis, prognosis, monitoring and risk assessment of patients with disturbances of the gastrointestinal tract activity and/or function and/or disturbances of the nutritional condition.

### Background of the invention

Somatostatin (SST, also called SRIF) was first described as secreted product of the hypothalamus that functions as potent inhibitor of human growth hormone secretion (hGH) (Siler et al. 1973. J Clin Endocrinol Metab 37:632-634*).* Later it was discovered that somatostatin is synthesized in a number of tissues, e.g. within the central and peripheral nervous system, pancreas, gastrointestinal tract and to a lower amount in the thyroid, kidney, adrenal medulla, submandibular gland, prostate and placenta.

Human mature somatostatin is derived from a larger precursor molecule (preproSomatostatin 1-116 (preproSST 1-116; SEQ ID NO:1); proSomatostatin 1-92 (proSST 1-92; SEQ ID NO:2). The sequence is encoded by chromosome 3. After cleavage of the signal sequence (24 amino acids in length) the prohormone proSST 1-92 is further processed into the biologically active peptides Somatostatin-28 (SST-28;28 amino acids in length; proSST 65-92) or Somatostatin-14 (SST-14; 14 amino acids in length; proSST 79-92). The fragment SST-28 (1-12) is deduced from SST-28 (Benoit et al. 1990. Metabolism 39:22-25). Moreover, proSomatostatin 1-76, a peptide containing SST-28 (1-12) at its C-terminus has been identified (Benoit et al. 1990. Metabolism 39:22-25).

Mature somatostatin, namely SST-14 and SST-28, have very short half life times in plasma of about 1-3 minutes (Sheppard et al. 1979. J Clin Endocrinol Metab 48:50-53*;* Hildebrand et al. 1994. Eur J Clin Invest 24:50-56). Due to its instability, a reliable measurement of circulating SST-14 and SST-28 is very difficult to achieve.

The serum concentration of somatostatin is increased postprandial (Penman et al. 1981. Gastroenterology 81:692-699*;* Polonsky et al. 1983. J Clin Invest 71: 1514-1518)*.* Ensinck et al. showed that SST-28, but not SST-14, is increased after ingestion of a mixed meal as well as after ingestion of protein and fat, whereas fat is supposed to be the main stimulus (Ensinck et al. 1990. Gastroenterology 98:633-638*;* Ensinck et al. 1989. J Clin Invest 83: 1580-1589). In contrast, the concentration of SST-14 and SST-28 was unchanged after ingestion of carbohydrates (Ensinck et al. 1990. Gastroenterology 98:633-638). This difference in the release of SST-14 and SST-28 stimulated by a mixed meal and pure glucose could be induced by a selective stimulation of cells from stomach, pancreas and intestinal D-cells, as SST-28 is the main product of the intestinal mucosa, whereas SST-14 is predominant in the pancreas (Baldissera et al. 1985. Biochem Biophys Acta 838:132-143).

Somatostatin regulates gastrointestinal motility via inhibition of gastric emptying, inhibits the contraction of the gall bladder and has a function as neurotransmitter within the central nervous system. Somatostatin is involved in the inhibition of insulin, glucagon, gastrin, ghrelin (Norrelund et al. 2002. Clin Endocrinol (Oxf.) 57: 539-546), secretin, cholecystokinin and the vasoactive intestinal peptide (VIP), norepinephrine, TRH (TSH releasing hormone), TSH, ACTH and CRH (corticotropin releasing hormone), gastric acid and pepsin.

It was shown that an N-terminal fragment of proSST (proSomatostatin 1-64, NT-proSST) is present in rat Aron et al. 1984. Biochem Biophys Res Comm 124:450-456*;* Aron et al. 1986. Endocrinology 118:218-222 and human (Aron et al. 1986. J Clin Endocrinol Metab 62:1237-1242) medullary thyroid carcinoma cells. In addition, proSST 1-64 was identified in the pancreas, the small intestinal mucosa and in plasma of pigs (Bersani et al. 1989. J Biol Chem 264:10633-10636; Holst et al. 1988. Pancreas 3:653-661*;* Skak-Nielsen et al. 1987. Regul Pept 19:183-195). Rabbani and colleagues identified rat proSST 1-64 as a secretion product in portal blood and in medium from cultured islet tumor cells (Rabbani and Patel 1990. Endocrinology 126:2054-2061). In plasma of patients with somatostatinoma, proSST 1-64 was found in high concentrations and therefore this molecule was discussed as possible marker for the diagnosis of somatostatinoma (Holst et al. 1991. The endocrine pancreas. New York: Raven Press, page 125-152). Somatostatinoma is a malignant tumor of the pancreas or duodenum producing somatostatin.

An immunohistochemical investigation in the rat, using a well-characterized antiserum raised against a synthetic peptide identical to the amino acid residues 20-36 of the proSST molecule, revealed the presence of immunoreactive nerve fibers and nerve terminal in specific areas of the rat brain (Mikkelsen et al. 1991. Neuroendocrinology 54:469-476). Extracts of posterior pituitaries from healthy rats subjected to size exclusion chromatography and RP-HPLC analysis showed the presence of a single proSST immunoreactive molecule corresponding to the size of proSST 1-64. The authors stated that its location in the hypothalamo-hypophyseal system suggests that this end product of the processing of proSST is released into the portal and perhaps also the general circulation. There is no known function of proSST 1-64. Fragments of proSST 1-64 have also been identified: proSST 1-10 (called antrin) (Benoit et al. 1987. Science 238:1126-1129) and proSST 1-32 in the intestine of pigs (Schmidt et al. 1985. FEBS Lett 192:141-146) as well as in extracts of the gastric and intestinal mucosa and plasma of rats *(*Ravazzola et al. 1989. J Clin Invest 83:362-366). Moreover, the existence of N-terminal proSST 1-63 from human pancreatic tumor was reported (Conlon et al. 1987. Biochem J 248:123-127). In addition, it has been shown that a 7-kDa peptide fragment of the N-terminal part of the proSST molecule, suggested to be equivalent to proSST 1-63 or proSST 1-64, is present in high concentrations in the rat nervous system (Patel et al. 1988. J Biol Chem 263:745-751*;* Rabbani and Patel 1990. Endocrinology 126:2054-2061), and immunohistochemically, Lechan et al. (Lechan et al. 1983. Proc Natl Acad Sci USA 80: 2780-2784) have demonstrated proSST 43-57-immunoreactive neurons in the rat brain, which may contain the same Pro-SST fragment. Furthermore, Odum and Johnston have described a human proSomatostatin fragment 29-92 in seminal fluid, containing the full mature SST at its C-terminus (Odum and Johnston 1994. Biochem J 303(Pt1):263-268).

A radioimmunoassay for the determination of proSST 20-36 immune reactivity was described by Bersani et al. (Bersani et al. 1989. J Biol Chem 264:10633-10636). By means of the antiserum used in that assay, proSST 1-64 was isolated from pancreas and intestinal mucosa. Aron et al. synthesized a tetradecapeptide containing the first 13 amino acids of rat proSST, to develop a radioimmunoassay directed toward the amino-terminal portion of prosomatostatin (Aron et al. 1984. Biochem Biophys Res Comm 124:450-456).

Plasma levels of mature somatostatin were investigated in gastrointestinal tract diseases with conflicting results most likely due to insufficient detection methods based on the instability of mature somatostatin as described above. Even though these results can actually not be evaluated, we briefly summarize the findings described in the literature. In patients with obstipation with delayed transit the basal SST concentration is unchanged, whereas the postprandial increase in plasma SST is pro-longated (Peracchi et al. 1999. Scand J Gastroenterol 34:25-28). Patients with inflammatory bowel diseases (including M. Crohn and Colitis ulcerosa) showed decreased SST-levels in intestinal mucosal tissue that was correlated with the histological grade of inflammation (Yamamoto et al. 1996. J Gastroenterol 31:525-532). A decrease in SST production was observed in cell cultures of colonic mucosa from patients with active colitis ulcerosa, whereas the generation of somatostatin by colonic mucosa of patients in remission was not significantly different from that of controls (Eliakim et al. 1991. Gastroenterology 100:A578). However, as these changes in SST production are localized to colonic mucosa, no conclusions can be drawn for the effect on circulating SST concentrations. On the contrary, in patients with colitis ulcerosa in the active phase the circadian (24 hour) rhythmicity of mature plasma somatostatin was found to have a higher 24-hour amplitude, a higher average level, and a longer peak level phase compared to healthy subjects (Payer et al. 1994. Hepatogastroenterology 41:552-553). The number of SST-containing endocrine mucosal and submucosal cells were significantly decreased in patients with Crohn's disease and colitis ulcerosa when compared to healthy controls, that was also related to the degree of inflammation (Watanabe et al. 1992. Dis Colon Rectum 35:488-494). Koch et al. demonstrated a decrease of SST in the mucosa of descending colon in patients with Crohn's disease and ulcerative colitis (Koch et al. 1988. Dis Colon Rectum 31:198-203). However, neither Watanabe et al. nor Koch et al. investigated somatostatin plasma levels. No difference in plasma SST was found in patients with chronic pancreatitis (El-Eryani et al. 2000. Hepatogastroenterology 47:869-874), whereas increased concentrations were found in patients with acute pancreatitis (Aleryani et al. 1997. Vnitr Lek 43:733-737). Milutinovic and colleagues demonstrated that plasma concentrations of SST were decreased in Helicobacter pylori infected gastritis patients and successful therapy resulted in an increase of SST concentrations in these patients (Milutinovic et al. 2003. European J Gastroenterol Hepatol 15:755-766). A significant difference in the 24-hour somatostatin level was revealed in patients with gastric ulcer and atypical gastric conditions, which was higher in patients with atypical conditions (Payer et al. 1995. Vntr Lek 41:367-370*).* The circadian rhythm of plasma somatostatin was studied in patients suffering from large bowel polyps, demonstrating significant differences (e.g. higher 24 hour amplitude) (Payer et al. 1995. Hemato-gastroenterology 42:775-777).

Somatostatin was also investigated in patients with cancer, again with conflicting results. Waisberg et al. have described a neuroendocrine gastric carcinoma (very malign, but rare type of tumor) showing the expression of SST (Waisberg et al. 2006. World J Gastroenterol 12:3944-3947). In addition, case-reports of SST-producing islet-cell tumors (pancreatic tumors) were described with increased concentrations of plasma SST (Karasawa et al. 2001. Intern Med 40:324-330*;* Howard et al. 1989. Surgery 105:227-229*;* Patel et al. 1983. J Clin Endocrinol Med 57:1048-1053*;* Permert et al. 1997. Pancreas 15:60-68). SST was discussed in the literature as plasma marker for the diagnosis of medullary thyroid carcinoma with conflicting results, showing an increase in SST (Roos et al. 1981. J Clin Endocrinol Med 52:187-194*;* Saito and Saito 1982. Horm Metab Res 14:71-76*;* Pacini et al. 1989. Cancer 63:1189-1195), no difference (Neradilova et al. 1989. Oncology 46:378-380) or a slight but non-significant decrease (Grauer et al. 1995. Thyroid 5:287-291). Wood and colleagues reported an increase of SST in some cases from patients with bronchial carcinoma, whereas no SST- concentration was detectable in the plasma (Wood et al. 1982. J Clin Endocrin Metab 53:1310-1312). In patients with small lung cell carcinoma plasma levels of SST were slightly increased (Noseda et al. 1987. Thorax 42:784-789). El-Salhy et al. demonstrated that the number of cells producing SST is reduced in colon tissue of patients with colon carcinoma (El-Salhy et al. 1998. Eur J Gastroenterol Hepatol 10:517-522). Plasma SST levels over a 24 hour period in patients suffering from large bowel cancer were compared with healthy subjects and patients suffering from other large bowel diseases (ulcerative colitis, large bowel polyps), showing no significant differences between patient groups in 24-hour SST secretion (Payer et al. 1997. Hepatogastroenterology 44:72-77). Holst et al. detected high concentrations of proSST 1-64 in plasma of patients with somatostatinoma and suggested this molecule as possible marker for the diagnosis of somatostatinoma. (Holst et al. 1991. The endocrine pancreas. New York: Raven Press, page 125-152). Even though high concentrations of proSST 1-64 were detected in these patients, the concentration of mature SST-14 was shown to be only slightly elevated, most likely due to the extremely short half-life time of SST-14. These results clearly demonstrate, that a reliable measurement of mature SST is extremely difficult to perform and Somatostatin concentrations reported in the literature have to be carefully evaluated.

A circadian rhythm was also described for mature Somatostatin (Payer et al. 1997. Hepatogastroenterology 44:72-77*;* Strazzulla et al. 1990. Chronobiologia 17:219-225*;* Payer et al. 1994. Hepatogastroenterology 41:552-553).

Autoantibodies against somatostatin and somatostatin-secreting cells were reported in patients with diabetes and GI-tract diseases (Bottazzo et al. 1976. Lancet 2:873-876*;* Jones et al. 1983. Gut 24:427-432).

WO 00/22439 A2 discloses proSomatostatin for the diagnosis of sepsis and other infections.

### Summary of the invention

A primary subject of the invention is a method for the diagnosis, prognosis, monitoring and risk assessment of disturbances of the gastrointestinal tract activity and/or function and/or disturbances of the nutritional condition in a patient comprising the steps of:
i) providing a sample of a bodily fluid of a patient,
ii) determining the level of proSomatostatin 1-64 or fragments thereof in said sample,
iii) correlating the level of proSomatostatin 1-64 or fragments thereof to the diagnosis, prognosis, monitoring and risk assessment of disturbances of the gastrointestinal tract activity and/or function and/or disturbances of the nutritional condition in said patient,
wherein said fragments have a length of at least 6 amino acid residues.

Preferred method variants are described in the dependent claims. The invention also relates to antibodies and a kit containing these antibodies.

### Description of drawings

The invention will now be described in more detail with reference to the drawings, which show:
- **Fig. 1**:: Correlation of polyclonal antibody-combination A and B
- **Fig. 2**:: Correlation of polyclonal antibody-combination A and C
- **Fig. 3**:: Correlation of polyclonal antibody-combination B and C
- **Fig. 4**:: Dose-response curve of the immunoassay with monoclonal antibodies
- **Fig. 5**:: Ex vivo stability of the proSST analyte
- **Fig. 6**:: Normal distribution of proSST in n=201 EDTA-plasma samples from healthy fasting subjects
- **Fig. 7**:: Correlation between proSST levels and Body Mass Index (BMI)
- **Fig. 8**:: Influence of food ingestion (mixed meal) on proSST levels
- **Fig. 9**:: ProSST concentrations in patients with disturbances of the gastrointestinal tract activity and/or function and/or disturbances of the nutritional condition.

### Detailed description of the invention

A primary subject of the invention is a method for the diagnosis, prognosis, monitoring and risk assessment of disturbances of the gastrointestinal tract activity and/or function and/or disturbances of the nutritional condition in a patient comprising the steps of:
(i) providing a sample of a bodily fluid of a patient,
(ii) determining the level of proSomatostatin 1-64 or fragments thereof in said sample,
(iii) correlating the level of proSomatostatin 1-64 or fragments thereof to the diagnosis, prognosis, monitoring and risk assessment of disturbances of the gastrointestinal tract activity and/or function and/or disturbances of the nutritional condition in said patient,
wherein said fragments have a length of at least 6 amino acid residues.

As used herein, human gastrointestinal (GI) tract refers to all the structures from the mouth to the anus. The GI-tract consists of the upper and lower GI tracts. The upper gastrointestinal tract consists of the esophagus, stomach, and duodenum. The lower gastrointestinal tract includes most of the small intestine and all of the large intestine. The small intestine consists of the three parts duodenum, jejunum and ileum, whereas the large intestine is divided into the cecum, colon and rectum.

In the context of the invention, disturbances of the human gastrointestinal tract activity and/or function and/or disturbances of the nutritional condition are the result of pathologically changed structures and/or functions of the GI tract or pathological changes of the nutritional condition. Disorders of the GI tract that affect the activity and/or function of the GI tract and/or disturbances of the nutritional condition include inflammatory, infectious, structural and functional GI-tract diseases.

Gastrointestinal tract infections, including the most common form gastroenteritis, can be caused by viruses, by bacteria (such as *Salmonella, Shigella, Campylobacter,* or *E. coli*), or by intestinal parasites (such as amebiasis and giardiasis).

Gastroenteritis (also known as gastric flu or stomach flu, although unrelated to influenza) is inflammation of the gastrointestinal tract, involving both the stomach and the small intestine and resulting in acute diarrhea and vomiting. It can be transferred by contact with contaminated food and water. The inflammation is caused most often by an infection from certain viruses or less often by bacteria, their toxins (e.g. SEB), parasites, or an adverse reaction to something in the diet or medication. At least 50 % of cases of gastroenteritis due to foodborne illness are caused by norovirus. Another 20 % of cases, and the majority of severe cases in children, are due to rotavirus. Other significant viral agents include adenovirus and astrovirus. Different species of bacteria can cause gastroenteritis, including *Salmonella, Shigella, Staphylococcus, Campylobacter jejuni, Clostridium, Escherichia coli, Yersinia, Vibrio cholerae,* and others. Gastroenteritis often involves stomach pain or spasms, diarrhea and/or vomiting, with noninflammatory infections of the upper small bowel, or inflammatory infections of the colon. The condition is usually of acute onset, normally lasting 1-6 days, and is self-limiting.

Gastrointestinal inflammatory diseases include inflammatory bowel disease (IBD) like Crohn's disease and ulcerative colitis; pancreatitis, appendicitis and coeliac disease.

Inflammatory bowel disease (IBD) is a group of inflammatory conditions of the colon and small intestine. The major types of IBD are Crohn's disease and ulcerative colitis. Other forms of IBD include collagenous colitis, lymphocytic colitis, ischemic colitis, diversion colitis, Behcet's disease and indeterminate colitis.

Crohn's disease, also known as regional enteritis, is an inflammatory disease of the intestines that may affect any part of the gastrointestinal tract from mouth to anus, causing a wide variety of symptoms. It primarily causes abdominal pain, diarrhea (which may be bloody if inflammation is at its worst), vomiting, or weight loss, but may also cause complications outside the gastrointestinal tract such as skin rashes, arthritis, inflammation of the eye, tiredness, and lack of concentration (Baumgart and Sandborn. 2007. The Lancet 369:1641-1657). Crohn's disease is thought to be an autoimmune disease, in which the body's immune system attacks the gastrointestinal tract, causing inflammation. It typically manifests in the gastrointestinal tract and can be categorized by the specific tract region affected. A disease of both the ileum (the last part of the small intestine, which connects to the large intestine), and the large intestine, ileocolic Crohn's accounts for fifty percent of cases.

Ulcerative colitis (Colitis ulcerosa) is a form of inflammatory bowel disease (IBD). Ulcerative colitis is a form of colitis, a disease of the intestine, specifically the large intestine or colon, that includes characteristic ulcers, or open sores, in the colon. The main symptom of active disease is usually constant diarrhea mixed with blood, of gradual onset. Ulcerative colitis, that has similarities to Crohn's disease, is an intermittent disease, with periods of exacerbated symptoms, and periods that are relatively symptom-free. Although the symptoms of ulcerative colitis can sometimes diminish on their own, the disease usually requires treatment to go into remission. Patients with ulcerative colitis are at an increased risk for the development of colorectal cancer (Kulaylat and Dayton. 2010. j Surg Oncol 101: 706-712).

Pancreatitis is an inflammation of the pancreas that can occur in two very different forms. Acute pancreatitis is sudden while chronic pancreatitis is characterized by recurring or persistent abdominal pain with or without steatorrhea or diabetes mellitus. The diagnostic criteria for pancreatitis are two of the following three features: 1) abdominal pain characteristic of acute pancreatitis, 2) serum amylase and/or lipase ≥ 3 times the upper limit of normal, and 3) characteristic findings of acute pancreatitis on CT scan (Banks and Freeman 2006. Am J Gastroenterol 101:2379-2400).

Appendicitis is a condition characterized by inflammation of the appendix. It is classified as a medical emergency and many cases require removal of the inflamed appendix, either by laparotomy or laparoscopy. Untreated, mortality is high, mainly because of peritonitis and shock (Hobler 1998. The Permanente Journal 2:5-8).

Coeliac disease is an autoimmune disorder of the small intestine that occurs in genetically predisposed people of all ages from middle infancy onward. Symptoms include chronic diarrhoea, failure to thrive (in children), and fatigue, but these may be absent, and symptoms in other organ systems have been described. Coeliac disease is caused by a reaction to gliadin, a prolamin (gluten protein) found in wheat, and similar proteins found in the crops of the tribe Triticeae (which includes other common grains such as barley and rye). Upon exposure to gliadin, and specifically to three peptides found in prolamins, the enzyme tissue transglutaminase modifies the protein, and the immune system cross-reacts with the small-bowel tissue, causing an inflammatory reaction. That leads to a truncating of the villi lining the small intestine (called villous atrophy). This interferes with the absorption of nutrients, because the intestinal villi are responsible for absorption. The only known effective treatment is a lifelong gluten-free diet (Sabatino and Corazza 2009. Lancet 373: 1480-1493). While the disease is caused by a reaction to wheat proteins, it is not the same as wheat allergy. This condition has several other names, including: coeliac disease, c(o)eliac sprue, non-tropical sprue, endemic sprue, gluten enteropathy or gluten-sensitive enteropathy, and gluten intolerance.

Structural disorders are those in which parts of the GI tract represent structural abnormalities, including diverticulitis, peptic ulcer and cancer.

Diverticulitis is a common digestive disease particularly found in the large intestine. Diverticulitis develops from diverticulosis, which involves the formation of pouches (diverticula) on the outside of the colon (Bogardus 2006. J Clin Gastroenterol 40 Suppl 3: S108-S111)*.* Diverticulitis results if one of these diverticula becomes inflamed. Patients often present with the classic triad of left lower quadrant pain, fever, and leukocytosis (an elevation of the white cell count in blood tests). Patients may also complain of nausea or diarrhea; others may be constipated.

Gastrointestinal cancer refers to malignant conditions of the gastrointestinal tract, including the esophagus, salivary gland, stomach, liver, biliary system, pancreas, bowels, colon and anus.

Neo-carcinoma is defined as new growth of an existing tumor.

Peptic ulcer or peptic ulcer disease, is an ulcer (defined as mucosal erosions equal to or greater than 0.5 cm) of an area of the gastrointestinal tract that is usually acidic and thus extremely painful. As many as 70-90 % of ulcers are associated with *Helicobacter pylori,* a spiral-shaped bacterium that lives in the acidic environment of the stomach; however, only 40 % of those cases go to a doctor. Ulcers can also be caused or worsened by drugs such as aspirin, Plavix (clopidogrel), ibupro-fen, and other NSAIDs. Contrary to general belief, more peptic ulcers arise in the duodenum (first part of the small intestine, just after the stomach) rather than in the stomach. About 4 % of stomach ulcers are caused by a malignant tumor, so multiple biopsies are needed to exclude cancer. Duodenal ulcers are generally benign.

Functional GI-tract disorders (FGID) include a number of separate idiopathic disorders which affect different part of the gastrointestinal tract. FGIDS are defined by chronic abdominal symptom complexes that occur in the absence of underlying structural abnormalities. The FGIDs, according to the Rome Criteria (http://www.romecriteria.org; Gastroenterology 2006. Volume 130 (No. 5): 1377-1552), include 6 major disorders in adults: functional esophageal disorders (e.g. functional heartburn, functional dysphagia), functional gastroduodenal disorders (e.g. functional dyspepsia, belching disorders, nausea and vomiting disorders), functional bowel disorders (e.g. irritable bowel syndrome [IBS]), functional abdominal pain syndrome, functional gallbladder and sphincter of oddi disorders, and functional anorectal disorders.

Irritable bowel syndrome (IBS or spastic colon) is a functional bowel disorder characterized by chronic abdominal pain, discomfort, bloating, and alteration of bowel habits in the absence of any detectable organic cause *(*Mayer 2008. NEJM. 358 : 1692-1699). In some cases, the symptoms are relieved by bowel movements. Diarrhea or constipation may predominate, or they may alternate. IBS may begin after an infection (post-infectious), a stressful life event, or onset of maturity without any other medical indicators.

Dyspepsia, also known as *upset stomach* or *indigestion,* refers to a condition of impaired digestion. It is a medical condition characterized by chronic or recurrent pain in the upper abdomen, upper abdominal fullness and feeling full earlier than expected when eating. It can be accompanied by bloating, belching, nausea, or heartburn. Dyspepsia is a common problem, and is frequently due to gastroesophageal reflux disease (GERD) or gastritis, but in a small minority may be the first symptom of peptic ulcer disease (an ulcer of the stomach or duodenum) and occasionally cancer. Functional dyspepsia is dyspepsia "without evidence of an organic disease that is likely to explain the symptoms" and is estimated to affect about 15 % of the general population in western countries (Saad and Chey 2006. Aliment. Pharmacol. Ther. 24: 475-92).

Dysphagia is the medical term for the symptom of difficulty in swallowing. Functional dysphagia is defined as having no organic cause for dysphagia that can be found.

In a preferred embodiment of the invention the diagnosis, prognosis and risk assessment of patients with inflammatory bowel disease (including Crohn's disease and ulcerative colitis), diverculitis, gastroenteritis, irritable bowel syndrome, pancreatitis, appendicitis, peptic ulcer disease, celiac disease and GI-tract carcinoma is carried out by the detection of proSomatostatin 1-64 and fragments thereof in biological samples.

The term nutritional condition is defined as the state of the body in relation to the consumption and utilization of nutrients and the ability of those nutrients to maintain normal metabolic integrity.

Somatostatin is physiologically associated with several regulatory processes of the gastrointestinal tract (e.g. the regulation of gastrointestinal motility via inhibition of several hormones). It was demonstrated by the inventors that pathologies, for which subnormal levels of proSST were measured, share the common characteristic of a reduced activity and/or function of the GI-tract, that may be directly or indirectly caused by the pathology. Surprisingly the activity and/or function of the GI-tract and/or the nutritional condition is the sole or at least dominating parameter, with which plasma levels of proSST is associated, and it is even more surprising that proSST levels are subnormal, when the activity and/or function of the Gl-tract and/or the nutritional condition is impaired.

In one embodiment of the invention, at least one further precursor peptide or fragment thereof related to the GI-tract is determined in addition to proSomatostatin 1-64 or fragments thereof, selected from the group consisting of ghrelin, cholecystokinin, gastrin, motilin, peptide YY, pancreatic polypeptide, secretin, glucagon, vasoactive intestinal peptide, gastric inhibitory peptide (GIP), amylin, neurotensin, substance P, gastrin releasing peptide (GRP) and bombesin.

In a preferred embodiment of the invention, proSomatostatin is measured in a sample from a fasting patient. Here, fasting is defined as abstinence from all food and drink except water for at least 8 hours.

In a particular embodiment of the invention, additionally at least one clinical parameter is determined selected from the group comprising: age, gender, body mass index (BMI), presence of diabetes mellitus and current smoking habits.

Body mass index (BMI) is defined as the individual's body weight divided by the square of his or her height. The formulae universally used in medicine produce a unit of measure of kg/m². The WHO regard a BMI of less than 18.5 as underweight and may indicate malnutrition, an eating disorder, or other health problems, while a BMI greater than 25 is considered overweight and above 30 is considered obese, a number above 40 suggests the person is morbidly obese (WHO 2000. Technical Report Series 894. Geneva: World Health Organization, 2000).

In a preferred embodiment of the invention the N-terminal proSomatostatin fragment comprising amino acids 1-64 (SEQ ID NO:6) of proSomatostatin (SEQ ID NO:2) is measured.

As mentioned herein in the context of pro-hormones like proSomatostatin, the term "fragment" refers to smaller proteins or peptides derivable from larger proteins or peptides, which hence comprise a partial sequence of the larger protein or peptide. Said fragments are derivable from the larger proteins or peptides by saponification of one or more of its peptide bonds. Such fragments are preferably detectable with immunological assays as described herein.

Determination of the level of proSomatostatin 1-64 and fragments thereof in a biological sample can be carried out using any suitable analytical technique known in the art. Such methods include but are not limited to immunoassays, mass spectrometry, immunoblot analysis, immunohistochemical methods, protein microarray methods and electrophoretic methods.

In a preferred embodiment of the invention the detection methods comprise immunoassays in various formats, such as for instance radioimmunoassay (RIA), chemiluminescence- and fluorescence- immunoassays, Enzyme-linked immunoassays (ELISA), Luminex-based bead arrays, protein microarray assays, and rapid test formats, such as for instance immunochromatographic strip tests.

The assays can be homogenous or heterogeneous assays, competitive and non-competitive assays. In a particularly preferred embodiment, the assay is in the form of a sandwich assay, which is a non-competitive immunoassay, wherein the molecule to be detected and/or quantified is bound to a first antibody and to a second antibody. The first antibody may be bound to a solid phase, e.g. a bead, a surface of a well or other container, a chip or a strip, and the second antibody is an antibody which is labeled, e.g. with a dye, with a radioisotope, or a reactive or catalytically active moiety. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford; 3rd ed. (May 2005), ISBN-13: 978-0080445267*;* Hultschig C et al., Curr Opin Chem Biol. 2006 Feb;10(1):4-10*. PMID: 16376134*, incorporated herein by reference).

In a particularly preferred embodiment the assay comprises two capture molecules, preferably antibodies which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.

Even more preferred, said labelling system comprises rare earth cryptates or rare earth chelates in combination with a fluorescence dye or chemiluminescence dye, in particular a dye of the cyanine type. In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, such as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethody-fluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzim-ides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in Kirk-Othmer, Encyclopedia of chemical technology, 4th ed., executive editor J. I. Kroschwitz; editor, M. Howe-Grant, John Wiley & Sons, 1993, vol.15, p. 518-562, incorporated herein by reference, including citations on pages 551-562. Preferred chemiluminescent dyes are acridiniumesters. Even more preferred, said labeling system comprises rare earth cryptates or rare earth chelates in combination with fluorescence dye.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

In the context of the present invention, "capture molecules" are molecules which may be used to bind target molecules or molecules of interest, i.e. analytes (i.e. in the context of the present invention the cardiovascular peptide(s)), from a sample. Capture molecules must thus be shaped adequately, both spatially and in terms of surface features, such as surface charge, hydrophobicity, hydrophilicity, presence or absence of lewis donors and/or acceptors, to specifically bind the target molecules or molecules of interest. Hereby, the binding may for instance be mediated by ionic, van-der-Waals, pi-pi, sigma-pi, hydrophobic or hydrogen bond interactions or a combination of two or more of the aforementioned interactions between the capture molecules and the target molecules or molecules of interest. In the context of the present invention, capture molecules may for instance be selected from the group comprising a nucleic acid molecule, a carbohydrate molecule, a PNA molecule, a protein, an antibody, a peptide or a glycoprotein. Preferably, the capture molecules are antibodies, including fragments thereof with sufficient affinity to a target or molecule of interest, and including recombinant antibodies or recombinant antibody fragments, as well as chemically and/or biochemically modified derivatives of said antibodies or fragments derived from the variant chain with a length of at least 12 amino acids thereof.

In another embodiment of the invention, the level of proSomatostatin 1-64 or fragments thereof is measured using mass spectrometric methods. The term mass spectrometry refers to methods of filtering, detecting and measuring ions based on their mass-to-charge ratio (or "m/z"). In general, mass spectrometry involves ionizing a sample containing one or more molecules of interest, and then n/z separating and detecting the resultant ions (or product ions derived therefrom) in a mass analyzer, such as a quadrupole mass filter, quadrupole ion trap, time-of-flight analyzer, FT/ICR analyzer or Orbitrap, to generate a mass spectrum representing the abundances of detected ions at different values of m/z.

In a preferred embodiment, the sample is subjected to one or more of the following steps prior to mass spectrometry: affinity enrichment of the analyte, enrichment for peptide fragments bound to carrier proteins; sample reduction, alkylation and/or desalting; one-dimensional SDS-polyacrylamide gel electrophoresis; trypsin proteolysis; or liquid chromatography.

The term level in the context of the present invention relates to the concentration (preferably expressed as weight/ volume; w/v) of marker peptides taken from a sample of a patient.

The term patient as used herein refers to a living human or non-human organism that is receiving medical care or that should receive medical care due to a disease. This includes persons with no defined illness who are being investigated for signs of pathology. Thus the methods and assays described herein are applicable to both, human and veterinary disease.

The term diagnosis in the context of the present invention relates to the recognition and (early) detection of a disease or clinical condition in a subject and also comprises differential diagnosis, risk stratification, prognosis, stratification for applying preventive and/or therapeutic measures and/or managements of patients, therapy monitoring, and therapy guidance of a disease or clinical condition.

Prognosis relates to the prediction of an outcome or a specific risk for a subject suffering from a particular disease or clinical condition. This may include an estimation of the chance of recovery or the chance of an adverse outcome for said subject.

In the present invention, the term risk stratification relates to the grouping of subjects into different risk groups according to their further prognosis. Risk stratification also relates to stratification for applying preventive and/or therapeutic measures.

The term sample as used herein refers to a sample of bodily fluid obtained for the purpose of diagnosis, prognosis, or evaluation of a subject of interest, such as a patient. Preferred test samples include blood, serum, plasma, cerebrospinal fluid, urine, saliva, sputum, and pleural effusions. In addition, one of skill in the art would realize that some test samples would be more readily analyzed following a fractionation or purification procedure, for example, separation of whole blood into serum or plasma components.
Thus, in a preferred embodiment of the invention the sample is selected from the group comprising a blood sample, a serum sample, a plasma sample, a cerebrospinal fluid sample, a saliva sample and a urine sample or an extract of any of the aforementioned samples. Preferably, the sample is a blood sample, more preferably a serum sample, and most preferably a plasma sample.
Where appropriate, the sample may need to be homogenized, or extracted with a solvent prior to use in the present invention in order to obtain a liquid sample. A liquid sample hereby may be a solution or suspension. Liquid samples may be subjected to one or more pre-treatments prior to use in the present invention. Such pre-treatments include, but are not limited to dilution, filtration, centrifugation, concentration, sedimentation, precipitation, dialysis. Pre-treatments may also include the addition of chemical or biochemical substances to the solution, such as acids, bases, buffers, salts, solvents, reactive dyes, detergents, emulsifiers, chelators.

The sensitivity and specificity of a diagnostic and/or prognostic test depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy) and "disease" populations (i.e. patients suffering from diabetes, insulin resistance and/or metabolic syndrome). For any particular marker, a distribution of marker levels for subjects with and without a disease will likely overlap. Under such conditions, a test does not absolutely distinguish normal from disease with 100 % accuracy, and the area of overlap indicates where the test cannot distinguish normal from disease. A threshold is selected, above which (or below which, depending on how a marker changes with the disease) the test is considered to be abnormal and below which the test is considered to be normal. The area under the ROC curve is a measure of the probability that the perceived measurement will allow correct identification of a condition. ROC curves can be used even when test results don't necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art (See, e.g., Hanley et al.1982. Radiology 143: 29-36). Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5 % of a given measurement.
The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve can be used to determine the effectiveness of the test.

In certain embodiments, markers and/or marker panels are selected to exhibit at least about 70 % sensitivity, more preferably at least about 80 % sensitivity, even more preferably at least about 85 % sensitivity, still more preferably at least about 90 % sensitivity, and most preferably at least about 95 % sensitivity, combined with at least about 70 % specificity, more preferably at least about 80 % specificity, even more preferably at least about 85 % specificity, still more preferably at least about 90 % specificity, and most preferably at least about 95 % specificity. In particularly preferred embodiments, both the sensitivity and specificity are at least about 75 %, more preferably at least about 80 %, even more preferably at least about 85 %, still more preferably at least about 90 %, and most preferably at least about 95 %. The term "about" in this context refers to +/- 5 % of a given measurement.

According to the method, the patient is diagnosed with having an increased probability of disturbances of the gastrointestinal activity and/or function and/or disturbances of the nutritional condition when said determined proSomatostatin level is lower than a predetermined threshold level. Preferably, the predetermined threshold level is below 400 pmol/L, more preferably between 300 pmol/L and 180 pmol/L, even more preferred between 250 pmol/L and 180 pmol/L, most preferred between 200 pmol/L and 180 pmol/L. In a preferred embodiment the patient is diagnosed of having disturbances of the gastrointestinal activity and/or function when said determined proSomatostatin level is lower than 400 pmol/L, preferably lower than 300 pmol/L, more preferably lower than 250 pmol/L, even more preferably lower than 200 pmol/L, most preferred lower than 180 pmol/L.

The above mentioned values might be different in other assays detecting proSomatostatin 1-64 or fragments thereof, if these have been calibrated differently. The above mentioned values shall apply for such differently calibrated proSST 1-64 assays accordingly, taking into account the differences in calibration. One possibility of quantifying the difference in calibration is a method comparison analysis (correlation) of the proSST 1-64 assay in question with the proSST 1-64 assay used in the present invention by measuring proSST 1-64 or fragments thereof in samples using both methods. Another possibility is to determine with the proSST 1-64 assay in question, given this test has sufficient analytical sensitivity, the median proSST 1-64 level of a representative normal population, compare results with the median proSST 1-64 levels as described here and recalculate the calibration based on the difference obtained by this comparison.

Preferably, the predetermined threshold level of proSomatostatin 1-64 or fragments thereof is below 112 % of the median of healthy fasting subjects, more preferably between 84 % and 50 %, even more preferred between 70 % and 50 %, most preferred between 56 % and 50 %. In a preferred embodiment the patient is diagnosed of having disturbances of the gastrointestinal activity and/or function when said determined level of proSomatostatin 1-64 or fragments thereof is lower than 112 % of the median of healthy fasting subjects, preferably lower than 84 %, more preferably lower than 70 %, even more preferably lower than 56 %, most preferred lower than 50 %.

Preferably, the predetermined threshold level of proSomatostatin 1-64 or fragments thereof is below the 63.5^{th} percentile of healthy fasting subjects, more preferably between the 30^{th} and the 0.25^{th} percentile, even more preferred between the 12.5^{th} and the 0.25^{th} percentile, most preferred the 2^{nd} and the 0.25^{th} percentile of healthy fasting subjects. In a preferred embodiment the patient is diagnosed of having disturbances of the gastrointestinal activity and/or function when said determined level of proSomatostatin 1-64 or fragments thereof is lower than the 63.5^{th} percentile of healthy fasting subjects, preferably lower than the 30^{th} percentile, more preferably lower than the 12.5^{th} percentile, even more preferably lower than the 2^{nd} percentile, most preferred lower than the 0.25^{th} percentile.

### Examples

### 1. Peptides

Four peptides related to proSST 1-64 were chemically synthesized, purified (HPLC; > 90% purity), and quality controlled (HPLC with a C18 column and absorbance measured at 220 nm) by JPT Peptide Technologies GmbH: Pro-SST 1-21 (PAK22; SEQ ID NO:3) with an additional Cystein residue at the C-terminus, Pro-SST 22-42 (PQD22; SEQ ID NO:4) with an additional Cystein residue at the N-terminus, Pro-SST 43-64 (PAR23; SEQ ID NO:5) with an additional Cystein residue at the N-terminus, and Pro-SST 1-64 (PAR64; SEQ ID NO:6).

### 2. Polyclonal antibodies

Polyclonal antibodies directed against the peptides PAK22, PQD22 and PAR23 were generated according to standard procedures. Using MBS (m-Maleimidobenzoyl-N-hydroxysuccinimid Ester) peptides were coupled to the carrier protein KLH (Keyhole limpet hemocyanin) (PIERCE, Rockford, IL, USA). Sheep were immunized with the peptide conjugates according to the following scheme: sheep were initially immunized with 100 µg conjugate (mass refers to the peptide moiety of the conjugate) and boostered thereafter in four-weekly intervals with 50 µg conjugate each time. Four months after the initial immunization 300 ml antiserum were obtained from each sheep. The peptides were covalently linked to Sulfolink coupling gel from Pierce (Boston, USA) according to the suppliers instructions and the respective polyclonal antibodies were purified from sheep antiserum by affinity purification. 50 ml of the corresponding sheep antiserum (anti PAK22 antiserum CF0784, anti PQD22 antiserum CF0788 and anti PAR23 antiserum CF0789) were incubated with the gels batchwise for 4 hours at room temperature. The materials were transferred in columns (empty NAP25 columns, Pharmacia). The flow through was discarded, the gels were washed with 100 ml wash buffer (100 mM K-phosphate, 0.1 % Tween 20, pH 6,8), and specifically bound antibodies were eluted with 50 mM citric acid, pH 2.7. The eluates were dialysed against 50 mM Na-phosphate, 100 mM NaCl, pH 8.0.

### 3. Monoclonal antibodies

Monoclonal antibodies directed against the peptides PAK22, PQD22 and PAR23 were generated by standard procedures (Harlow E, Lane D. Antibodies - A Laboratory Manual. Cold Spring Harbor: Cold Spring Harbor Laboratory, 1988*;* Lane 1985. J Immunol Methods 81:223-228.). Briefly, peptides were conjugated to BSA by using Sulfo-MBS (m-maleimidobenzoyl-N-hydroxysuccinimid Ester). With these conjugates Balb/c mice were immunized and boostered, and spleen cells were fused with SP2/0 myeloma cells to generate hybridoma cell lines. Cell lines were screened for their ability to secrete antibodies that would bind to the immunogenic peptides, which were coated on a solid polystyrene phase. With this approach, cell lines secreting monoclonal antibodies 313/1F11 (against PAK22), 314/1A11 (against PQD22) and 315/1A9 (against PAR23) were generated. For further experiments, monoclonal antibodies were purified from culture supernatant by Protein G affinity chromatography.

### 4. Coating and labeling of antibodies

Polystyrene startubes (Greiner) were coated with purified antibodies (2 µg antibody per tube in 300 µL of 50 mmol/L Tris, 100 mmol/L NaCl, pH 7.8) overnight at 22 °C. Tubes were blocked with 10 mmol/L Na-phosphate (pH 6.5) containing 3 % Karion FP (Merck), 0.5% BSA protease free (Sigma) and lyophilized.
The concentration of the purified antibodies was adjusted to 1 g/L, and the antibodies were labelled by incubation with the chemiluminescent label MACN-Acridinium-NHS-Ester (1 g/L; InVent GmbH, Hennigsdorf, Germany) in a 1:5 molar ratio for 20 min at room temperature. The reactions were stopped by addition of 1/10 volume of 1 mol/L Tris for 10 min. Labeled antibodies were separated from free label by size-exclusion chromatography on a NAP-5 column (GE Healthcare, Freiburg, Germany) and a Bio-Sil® SEC-400-5 HPLC column (BIO-RAD).

### 5. Immunoassays with polyclonal or monoclonal antibodies

Tracers were produced by diluting the respective labelled antibody in assay buffer (300 mmol/L potassium phosphate, 100 mmol/L NaCl, 10 mmol/L sodium EDTA, 5 g/L bovine serum albumin protease free (Sigma), 1 g/L nonspecific sheep IgG, 1 g/L nonspecific bovine IgG, 1 g/L nonspecific mouse IgG, 0.9 g/L sodium azide, pH 7.0) containing 10⁶ relative light units (RLU) of MACN-labeled antibody per 200 µl.
Dilutions of peptide PAR64 in null serum (T3/T4 free serum, non liped stripped from Scantibodies) served as calibrators with concentrations of 2560, 640, 160, 40, 10 pmol/L, respectively. The immunoassays were performed by incubating 50 µL of calibrator or patient sample and 200 µL of tracer under agitation for 2 h at room temperature (18 -27 °C). Tubes were washed 4 times with 1 mL of washing solution (B.R.A.H.M.S GmbH), and bound chemiluminescence was measured for 1 s per tube with a LB952T luminometer (Berthold).

### Results

### 1. Correlation of immunoassays with polyclonal antibodies

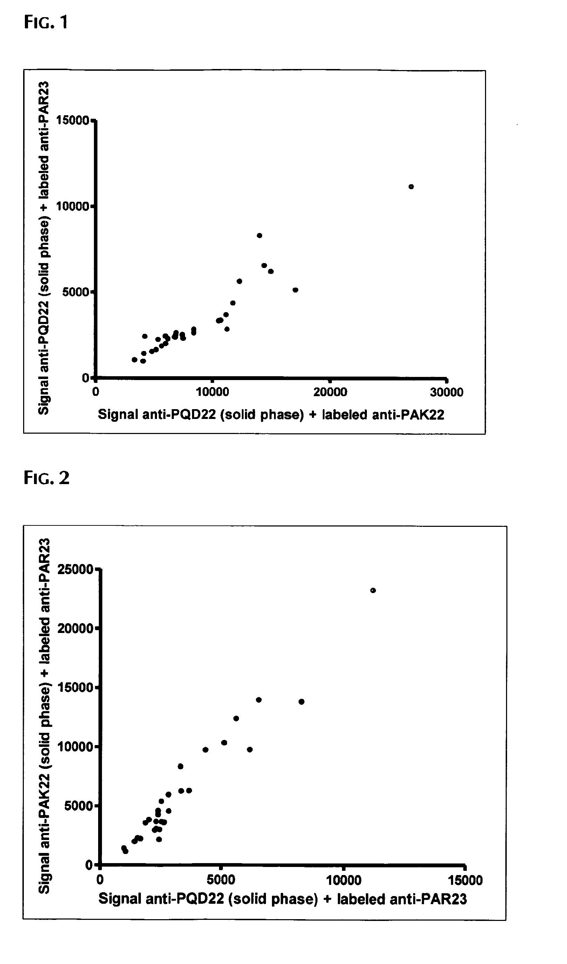

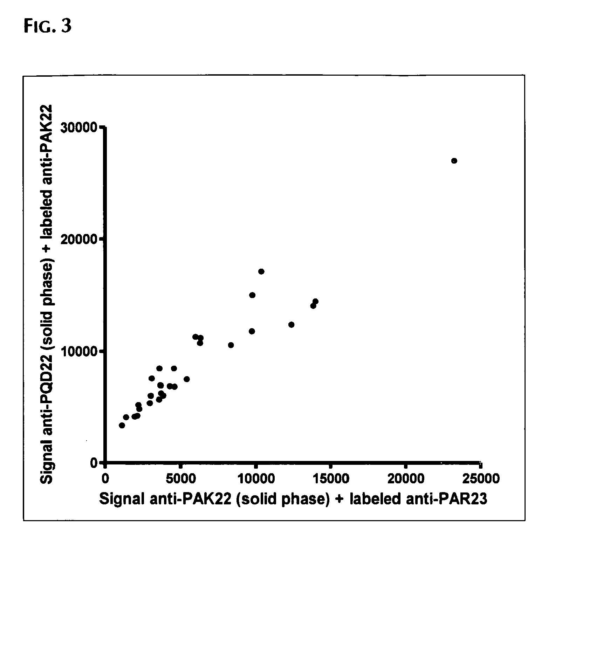

30 EDTA-plasma samples from patients (including healthy individuals and patients suffering from Crohn's disease, pancreatitis and different carcinomas) were measured in three immunoassay variants (solid-phase antibody anti-PQD22 + labelled anti-PAR23 antibody [combination A], solid-phase antibody anti-PQD22 + labelled anti-PAK22 antibody [combination B] and solid-phase antibody anti-PAK22 + labelled anti-PAR23 antibody [combination C]). Prosomatostatin immunoreactivity was detectable with all three antibody combinations. The correlation between each two combinations was analyzed, respectively. The correlation was r=0.94 between combination A and B (Fig. 1), r=0.91 between combination A and C (Fig. 2), and r=0.94 between combination B and C (Fig. 3), respectively.

### 2. Technical characterization of the immunoassay with monoclonal antibodies

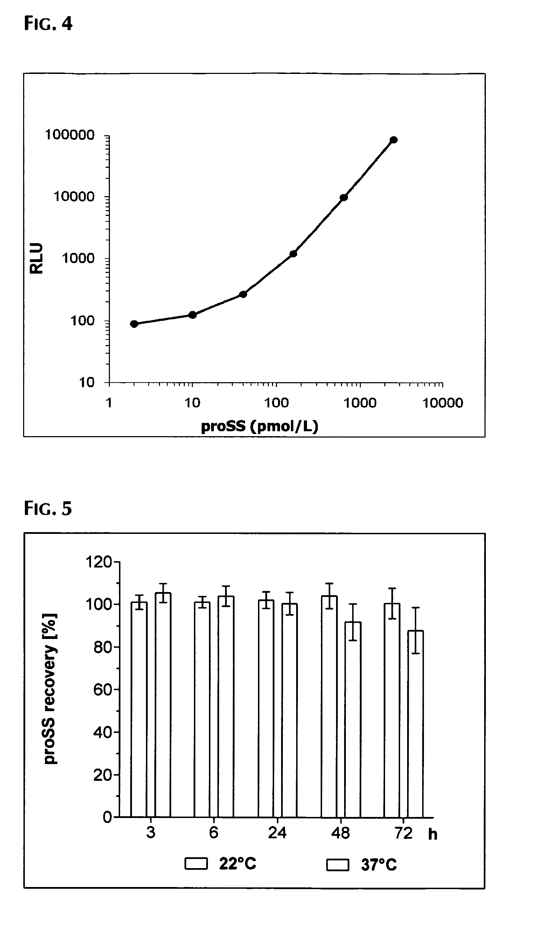

Six sandwich immunoassays using different combinations of antibodies were tested with a high PAR64 standard peptide concentration (5600 pmol/L) and two EDTA-plasma pools from patients with low and high proSST concentration, respectively. The combination with the highest measuring values (anti-PAK22 313/1F11 as solid phase and anti-PQD22 314/1A11 as labeled antibody) was used for all further experiments.
The lower limit of detection (LoD), as determined with T3/T4 free serum non liped stripped (mean relative light units of 10 determinations plus 2 SD), was 4 pmol/L. A high-dose hook effect was observed for PAR64 concentrations > 8300 pmol/L.
The total precision of the assay was determined by measuring 30 human EDTA-plasma samples. These data were generated by five different operators for 10 assay runs using one lot and two luminometers according to protocol EP-5A2 recommended by the Clinical and Laboratory Standards Institute CLSI (formerly NCCLS). The CV was < 20 % for measured samples with a proSST concentration > 25 pmol/L. Accuracy was assessed by dilution experiments. Linear dilutions with null serum (up to 1:16, with the 1:2 dilution prepared by mixing equal volumes of sample and diluent and the 1:4, 1:8 and 1:16 dilutions prepared by mixing a portion of the previous dilution with an equal volume of diluent) were tested in 5 EDTA-plasma samples. Measured concentrations were multiplied by the dilution factor and compared with the values for the undiluted samples. None of the 5 samples showed a deviation during dilution > 20 % of the original value.
A typical dose response curve using the synthetic peptide PAR64 as calibrator is shown in Figure 4.

### 3. Ex vivo stability of the analyte

We evaluated the stability of the native analyte at 22 °C and 37 °C in EDTA-plasma from 10 different individuals. At 22 °C the analyte was stable (< 10 % loss of immunoreactivity) for 72 h and at 37 °C for 24 h (see Figure 5). (Stability in serum is considerably lower; data not shown here). In 5 EDTA-plasma samples, freezing and thawing 4 times had no influence on the measured concentration of proSST (mean values, 99.1 % [range, 93.8 %-104.3 %] of the original values).

### 4. Clinical data

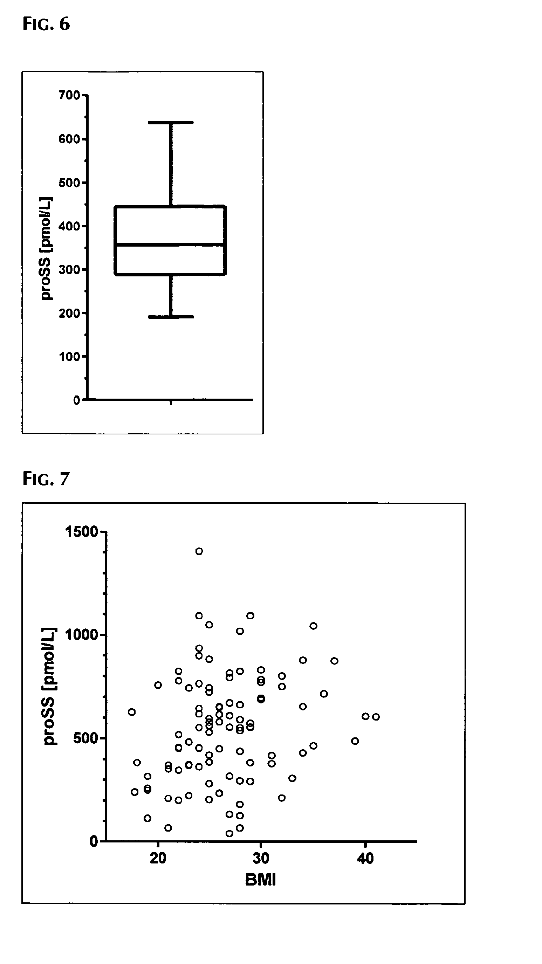

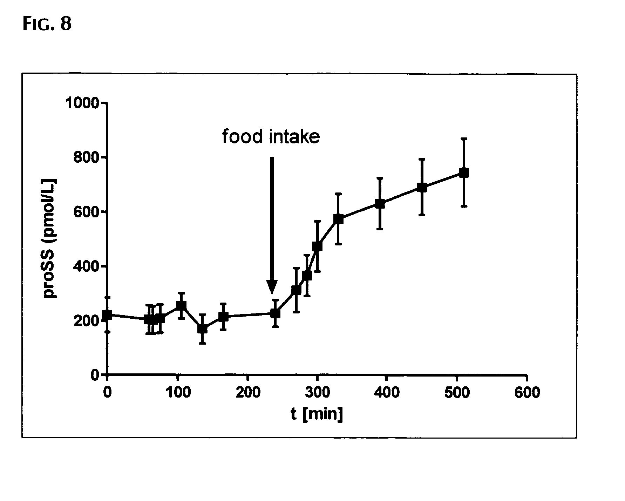

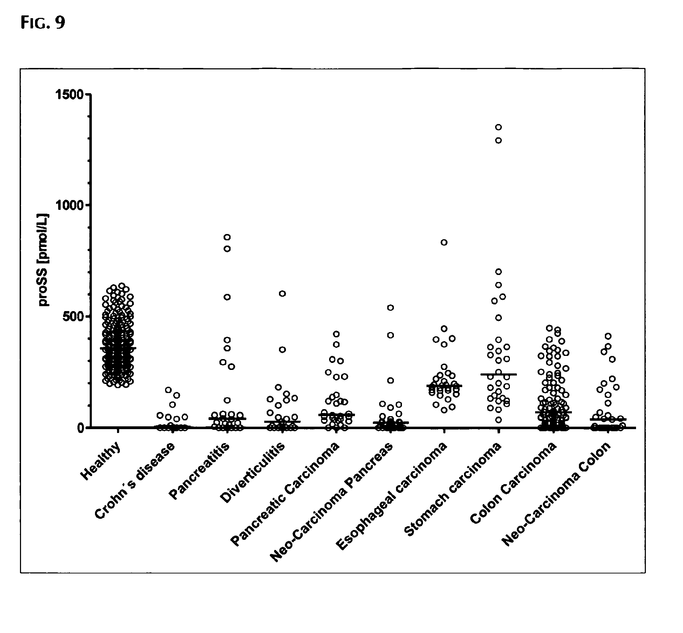

ProSST was measured in 201 EDTA-plasma samples from healthy fasting subjects with a median concentration of 357 pmol/L (range 191-637 pmol/L) (Figure 6). The 2.5^{th} percentile was 205 pmol/L, the 25^{th} percentile was 288 pmol/L, the 75^{th} percentile was 445 pmol/L and the 97.5^{th} percentile was 609 pmol/L, respectively.
In fasting subjects, an association of proSST levels with the Body Mass Index (BMI) was observed (Spearman rank correlation r=0.23 [P<0.05]) (Figure 7).
In another experiment six healthy subjects ingested a mixed meal after an overnight fasting period (for at least 8 hours). EDTA-plasma samples were serially gained and proSST was measured. The concentration of proSST increased significantly after the ingestion of food (Kruskal Wallis test P<0.0001) (Figure 8).
Samples from fasting patients suffering from different GI-tract diseases (e.g. inflammatory bowel diseases like Crohn's disease; pancreatitis; diverculitis; and different GI-tract carcinoma types) were measured and proSST concentrations were significantly decreased compared to healthy fasting controls (Kruskal Wallis test P<0.0001) (Figure 9). Receiver operating characteristics resulted in an area under curve (AUC) of 1.0 (95 % CI 1.0 - 1.0) for patients with Crohn's disease, 0.82 (95 % CI 0.69 - 0.95) for patients with pancreatitis, 0.94 (95 % Cl 0.86 - 1.02) for patients with diverculitis, 0.93 (95 % CI 0.87 - 0.99) for patients with pancreatic carcinoma, 0.95 (95 % Cl 0.87 - 1.02) for patients with pancreatic neo-carcinoma, 0.86 (95 % CI 0.77 - 0.96) for patients with esophageal carcinoma, 0.66 (95 % CI 0.52 - 0.80) for patients with carcinoma of the stomach, 0.92 (95 % CI 0.88 - 0.96) for patients with colon carcinoma, and 0.93 (95 % Cl 0.87 - 0.99) for patients with neo-colon carcinoma, respectively. Exemplary cut-off values with the resulting specificity and sensitivity are summarized for the different patient groups (Table 1).
The pathologies, for which subnormal levels of proSST were detected, share a common characteristic, that is a reduced activity/ function of the GI-tract, be it directly or indirectly caused by the pathology. As the physiological role of somatostatin in the prior art has been associated with several regulatory processes, it is surprising that apparently activity/ function of the GI-tract is the sole or at least dominating parameter, with which plasma levels of proSST is associated, and even more surprising that proSST levels are subnormal, when activity/ function of the GI-tract is impaired.

As it has been reported that autoantibodies against somatostatin and somatostatin-secreting cells might exist in patients with diabetes and GI-tract diseases (Bottazzo et al. 1976. Lancet 2:873-876*;* Jones et al. 1983. Gut 24:427-432), we addressed the question of whether possibly autoantibodies against proSST might exist and possibly influence measurement obtained in the described examples above. Accuracy of proSST measurement, assessed by dilution and recovery experiments known to persons skilled in the art, was ideal, when samples from patients with GI-tract diseases, other diseases and healthy individuals were analyzed. Thus, it can be excluded that the proSST measurements shown in the examples above are impaired or inaccurate, and apparently no anti-proSST autoantibodies exist, which interfere with the proSST measurements performed.

Sequences

SEQ ID NO:1

10 20 30 40 50 60
MLSCRLQCAL AALSIVLALG CVTGAPSDPR LRQFLQKSLA AAAGKQELAK YFLAELLSEP

70 80 90 100 110
NQTENDALEP EDLSQAAEQD EMRLELQRSA NSNPAMAPRE RKAGCKNFFW KTFTSC

SEQ ID NO:2

10 20 30 40 50 60
APSDPRLRQF LQKSLAAAAG KQELAKYFLA ELLSEPNQTE NDALEPEDLS QAAEQDEMRL

70 80 90
ELQRSANSNP AMAPRERKAG CKNFFWKTFT SC

SEQ ID NO:3

10 20
APSDPRLRQF LQKSLAAAAG K

SEQ ID NO:4

10 20
QELAKYFLAE LLSEPNQTEN D

SEQ ID NO: 5

10 20
ALEPEDLSQA AEQDEMRLEL QR

SEQ ID NO:6

10 20 30 40 50 60
APSDPRLRQF LQKSLAAAAG KQELAKYFLA ELLSEPNQTE NDALEPEDLS QAAEQDEMRL ELQR

### FIGURES AND TABLES

**Table 1: Exemplary cut-off values (with corresponding specificity and sensitivity values) for patient groups suffering from different Gl-tract diseases**

| **Patient group** | **Cut-off value (pmol/L)** | **Specificity (%)** | **Sensitivity (%)** |
|---|---|---|---|
| Crohn's disease | 180 | 100 | 100 |
| | 200 | 98.5 | 100 |
| | 250 | 87.6 | 100 |
| | 300 | 70.7 | 100 |
| | 400 | 36.8 | 100 |
| Pancreatitis | 180 | 100 | 72.0 |
| | 200 | 98.5 | 72.0 |
| | 250 | 87.6 | 72.0 |
| | 300 | 70.7 | 80.0 |
| | 400 | 36.8 | 88.0 |
| Diverculitis | 180 | 100 | 92.0 |
| | 200 | 98.5 | 92.0 |
| | 250 | 87.6 | 92.0 |
| | 300 | 70.7 | 92.0 |
| | 400 | 36.8 | 96.0 |
| Pancreatic carcinoma | 180 | 100 | 76.7 |
| | 200 | 98.5 | 76.7 |
| | 250 | 87.6 | 86.7 |
| | 300 | 70.7 | 90.0 |
| | 400 | 36.8 | 96.7 |
| Pancreatic neo-carcinoma | 180 | 100 | 90.0 |
| | 200 | 98.5 | 90.0 |
| | 250 | 87.6 | 93.3 |
| | 300 | 70.7 | 93.3 |
| | 400 | 36.8 | 93.3 |
| Esophageal carcinoma | 180 | 100 | 43.0 |
| | 200 | 98.5 | 63.3 |
| | 250 | 87.6 | 80.0 |
| | 300 | 70.7 | 83.3 |
| | 400 | 36.8 | 90.0 |
| Stomach carcinoma | 180 | 100 | 36.7 |
| | 200 | 98.5 | 43.3 |
| | 250 | 87.6 | 53.3 |
| | 300 | 70.7 | 53.3 |
| | 400 | 36.8 | 76.7 |
| Colon carcinoma | 180 | 100 | 75.0 |
| | 200 | 98.5 | 76.0 |
| | 250 | 87.6 | 83.0 |
| | 300 | 70.7 | 87.0 |
| | 400 | 36.8 | 97.0 |
| Neo-colon carcinoma | 180 | 100 | 76.7 |
| | 200 | 98.5 | 83.3 |
| | 250 | 87.6 | 86.7 |
| | 300 | 70.7 | 86.7 |
| | 400 | 36.8 | 96.7 |

## Claims

1. A method for the diagnosis, prognosis, monitoring and risk assessment of disturbances of the gastrointestinal tract activity and/or function and/or disturbances of the nutritional condition in a patient comprising the steps of:
i) providing a sample of a bodily fluid of a patient,
ii) determining the level of proSomatostatin 1-64 or fragments thereof in said sample,
iii) correlating the level of proSomatostatin 1-64 or fragments thereof to the diagnosis, prognosis and risk assessment of disturbances of the gastrointestinal tract activity and/or function and/or disturbances of the nutritional condition in said patient,
wherein said fragments have a length of at least 6 amino acid residues.

2. The method according to claim 1, wherein the sample of a bodily fluid is taken from said patient after a fasting period of at least 8 hours.

3. The method according to claims 1 and 2, wherein said patient is suffering from a gastrointestinal disease **characterized by** disturbances of the gastrointestinal tract activity and/or function and/or disturbances of the nutritional condition selected from the group comprising inflammatory, infectious, structural and functional gastrointestinal tract diseases.

4. The method according to claim 3, wherein said infectious gastrointestinal tract disease is caused by viruses, by bacteria, or by intestinal parasites.

5. The method according to claim 3, wherein said inflammatory gastrointestinal tract disease is selected from the group comprising inflammatory bowel disease, including Crohn's disease and ulcerative colitis; pancreatitis, appendicitis, and celiac disease.

6. The method according to claim 3, wherein said structural gastrointestinal tract disease is selected from the group comprising cancer, especially cancer of the esophagus, the salivary gland, the stomach, the liver, the biliary system, the pancreas, the bowels, the colon and the anus; diverticulitis and peptic ulcer disease.

7. The method according to claim 3, wherein said functional gastrointestinal tract disease is selected from the group comprising functional esophageal disorders (including functional heartburn, functional dysphagia), functional gastroduodenal disorders (including functional dyspepsia, belching disorders, nausea and vomiting disorders), functional bowel disorders (including irritable bowel syndrome [IBS]), functional abdominal pain syndrome, functional gallbladder and sphincter of oddi disorders, and functional anorectal disorders.

8. The method according to any one of claims 1 to 7, wherein said sample of a bodily fluid is selected from blood, serum, plasma, cerebrospinal fluid, urine or saliva.

9. The method according to claim 1 to 8, wherein at least one further marker peptide or precursor or fragment thereof related to the gastrointestinal tract is determined, preferably selected from the group comprising ghrelin, cholecystokinin, gastrin, motilin, peptide YY, pancreatic polypeptide, secretin, glucagon, vasoactive intestinal peptide, gastric inhibitory peptide (GIP), amylin, neurotensin, substance P, gastrin releasing peptide (GRP) and bombesin.

10. The method of any one of claims 1 to 9, wherein additionally at least one clinical parameter is determined selected from the group comprising age, gender, body mass index (BMI), presence of diabetes mellitus and current smoking habits.

11. The method according to any one of the preceding claims, wherein disturbances of the gastrointestinal tract activity and/or function and/or disturbances of the nutritional condition are diagnosed and predicted in a patient and/or patients with disturbances of the gastrointestinal tract activity and/or function and/or disturbances of the nutritional condition and are stratified to an increased risk when said determined level of proSomatostatin 1-64 or fragments thereof is lower than a predetermined threshold level.

12. The method according to claim 11, wherein the predetermined threshold level of proSomatostatin 1-64 or fragments thereof is below 400 pmol/L, preferably between 400 and 180 pmol/L, more preferably between 300 and 180 pmol/L, even more preferred between 250 pmol/L and 180 pmol/L, most preferred between 200 pmol/L and 180 pmol/L.

13. The method according to claim 11, wherein the predetermined threshold level of proSomatostatin 1-64 or fragments thereof is below 112 % of the median of healthy fasting subjects, more preferably between 84 % and 50 %, even more preferred between 70 % and 50 %, most preferred between 56 % and 50 %.

14. The method according to claim 11, wherein the predetermined threshold level of proSomatostatin 1-64 or fragments thereof is below the 63.5^{th} percentile of healthy fasting subjects, more preferably between the 30^{th} and the 0.25^{th} percentile, even more preferred between the 12.5^{th} and the 0.25^{th} percentile, most preferred the 2^{nd} and the 0.25^{th} percentile of healthy fasting subjects.

15. The method of any one of claims 1 to 14, wherein the level of proSomatostatin 1-64 is determined.

16. An antibody which binds to an epitope comprised in a peptide representing amino acids 1 to 21 of proSomatostatin (SEQ ID NO:2).

17. An antibody which binds to an epitope comprised in a peptide representing amino acids 22 to 42 of proSomatostatin (SEQ ID NO:2).

18. An antibody which binds to an epitope comprised in a peptide representing amino acids 43 to 64 of proSomatostatin (SEQ ID NO:2).

19. Kit for determining the level of proSomatostatin 1-64 or fragments thereof containing at least one antibody according to any one of claims 16 to 18.
